(19) Europäisches Patentamt
European Patent Office
Office européen des brevets

(11) Numéro de publication: **0 070 049 B1**

# (12) FASCICULE DE BREVET EUROPEEN

(45) Date de publication du fascicule du brevet: **05.12.84**

(51) Int. Cl.³: **C 07 C 69/90**, A 61 K 31/235

(21) Numéro de dépôt: **82200675.5**

(22) Date de dépôt: **02.06.82**

## (54) Dérivé de l'acide acétylsalicylique ayant activité analgésique, antipyrétique, antiphlogistique et bronchosécrétolytique.

(30) Priorité: **22.06.81 IT 6786481**

(43) Date de publication de la demande: **19.01.83 Bulletin 83/3**

(45) Mention de la délivrance du brevet: **05.12.84 Bulletin 84/49**

(84) Etats contractants désignés: **CH DE FR GB IT LI**

(56) Documents cités:
**DE - A - 2 538 206**
**FR - A - 2 270 860**
**FR - A - 2 441 606**

(73) Titulaire: **UNIONE CHIMICA MEDICAMENTI-DIFME-S.p.A., Via Sabaudia 44, I-10095 Grugliasco (Torino) (IT)**

(72) Inventeur: **Tedeschi, Sergio, Via Piffetti 47, I-10143 Torino (IT)**
Inventeur: **Spano, Remo, Dr., Via Condove 98, I-10093 Collegno (Torino) (IT)**

(74) Mandataire: **Patrito, Pier Franco, Dr. Ing., Via Don Minzoni 14, I-10121 Torino (IT)**

EP 0 070 049 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un nouveau dérivé de l'acide acétylsalicylique.

On sait que l'acide acétylsalicylique est amplement employé dans le traitement des affections fiévreuses, des maladies dérivant des rhumes, des affections rhumatismales et des affections névralgiques, en considération de son activité analgésique, antipyrétique et antiphlogistique. Cependant, cet acide présente des inconvénients et des dangers à cause de sa non négligeable toxicité qui se manifeste soit sous forme aiguë, soit sous forme subaiguë, spécialement en produisant des lésions gastriques, et, dans certains cas, il n'est pas bien toléré.

Dans certaines affections, et en particulier dans les affections catarrhales de l'arbre respiratoire sur base phlogistique, fiévreuse et douloureuse et dans les complications au niveau trachéo-broncho-pulmonaire, il est opportun d'associer une action mucolytique, et c'est donc pour cette raison que dans ces cas l'acide acétylsalicylique est administré en même temps qu'une substance ayant une activité bronchosécrétolytique, telle que par exemple les dérivés du gaïacol.

Le but de la présente invention est de réaliser une substance ayant une toxicité inférieure à celle de l'acide acétylsalicylique, une activité analgésique, antipyrétique et antiphlogistique supérieure à celle de l'acide acétylsalicylique, et en plus une activité bronchosécrétolytique comparable à celle des dérivés du gaïacol.

L'objet de l'invention est un nouveau dérivé de l'acide acétylsalicylique, constitué par acétylsalicylate de 2(o-méthoxyphénoxy)éthanol, ayant la formule structurale suivante:

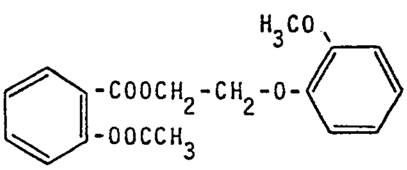

Ce dérivé de l'acide acétylsalicylique est indiqué pour être utilisé comme agent ayant une activité analgésique, antipyrétique, antiphlogistique et bronchosécrétolytique.

Il est indiqué pour être utilisé dans le traitement des affections fiévreuses, des maladies dérivant des rhumes, des affections rhumatismales et des affections névralgiques, et spécialement dans le traitement des affections catarrhales de l'arbre respiratoire sur base phlogistique, fiévreuse et douloureuse et des complications au niveau trachéo-broncho-pulmonaire.

L'acétylsalicylate de 2(o-méthoxyphénoxy)-éthanol peut être obtenu au moyen de méthodes chimiques d'estérification connues, par estérification du carboxyle de l'acide acétylsalicylique avec l'hydroxyle du 2(o-méthoxyphénoxy)éthanol. Afin de mieux éclairer l'invention, on va décrire maintenant un exemple, non limitatif, de procédé au moyen duquel on peut obtenir le produit faisant l'objet de la présente invention.

*Exemple de préparation:*

On prépare préalablement du 2(o-méthoxyphénoxy)éthanol en faisant réagir à reflux le gaïacol avec le 2-chloroéthanol en solution aqueuse d'hydroxyde de sodium. La méthode de préparation de cette solution est décrite en détail par K.S. Bukarev *et al.*, («Zhur. Obshchei Khim.», vol. 24, pp. 2014-2023, 1954).

Dans un ballon de verre muni d'un refroidisseur à reflux à gravité, d'un agitateur et d'un entonnoir à charger, on introduit 5,5 g de 2(o-méthoxyphénoxy)éthanol et 5 ml de triéthylamine. On réchauffe légèrement et successivement, on ajoute goutte à goutte 6,5 g d'acétylsalicyloïlchlorure dilués en 20 ml de chloroforme. On soumet le mélange à ébullition à reflux pour 3 h, on le refroidit et on lave la solution chloroformique avec deux portions d'eau de 50 ml chacune. On dessèche avec sulfate de sodium anhydre la solution chloroformique et on fait évaporer le solvant. Le résidu solide est repris avec de l'alcool éthylique à chaud et ensuite on le fait cristalliser par refroidissement, en obtenant ainsi l'acétylsalicylate de 2(o-méthoxyphénoxy)éthanol.

*Caractéristiques de la substance*

L'acétylsalicylate de 2(o-méthoxyphénoxy)-éthanol est une substance cristalline blanche, ayant une légère saveur et une odeur de gaïacol, soluble en benzène, chloroforme, diméthylcétone, éthanol, éther éthylique, isopropanol et autres solvants organiques, et pratiquement insoluble dans l'eau. Son point de fusion est entre 67 et 70° C, son poids moléculaire est de 330,34, sa formule brute est $C_{18}H_{18}O_6$ et sa formule structurale est celle idiquée auparavant. Soumis à la chromatographie en couche mince et utilisant comme diluant un mélange de 90% de benzène et 10% d'isopropanol, il présente une hauteur de montée Rf = 0,75 ÷ 0,78.

L'acétylsalicylate de 2(o-méthoxyphénoxy)-éthanol est doué d'activité antiphlogistique, antipyrétique, analgésique et bronchosécrétolytique. Dans le but de mettre en évidence ces activités, on a effectué des épreuves pharmacologiques comparatives par rapport à l'acide acétylsalicylique, pour ce qui concerne l'activité antiphlogistique, antipyrétique et analgésique et la toxicité, et par rapport au gaïacoléthanol, pour ce qui concerne l'activité bronchosécrétolytique. Les résultats de ces épreuves sont décrits ci-dessous.

*Activité antiphlogistique*

L'activité antiphlogistique de l'acétylsalicylate de 2(o-méthoxyphénoxy)éthanol a été évaluée en comparaison avec celle de l'acide acétylsalicylique sur la base de la variation de volume de l'œdème induit par carraghénine injectée dans des pattes de rats, suivant la technique décrite par Winter *et al.*, («Proc. Soc. Exp. Biol. Med.», vol. III, p. 544, 1962). Les résultats de l'élaboration des épreuves sont indiqués dans le tableau 1, dans lequel le volume de l'œdème est indiqué en millilitres ± la déviation standard, et il a été mesuré 3, 4,5 et 6 h

après le traitement œdémigène; la dose de substance est indiquée en milligrammes de substance administrée par voie orale pour chaque kilogramme de poids corporel des rats; les valeurs marquées par * correspondent à une probabilité d'erreur $p < 0,05$, les valeurs marquées ** correspondent à une probabilité d'erreur $p < 0,01$, calculées sur les valeurs des contrôles, et les valeurs indiquées entre parenthèses indiquent l'inhibition en pour-cent calculée par rapport aux contrôles.

De l'examen des résultats des épreuves, indiqués dans le tableau 1, on constate que l'acétylsalicylate de 2(o-méthoxyphénoxy)-éthanol est doué d'une activité antiphlogistique nettement supérieure à celle de l'acide acétylsalicylique.

*Activité antipyrétique*

L'activité antipyrétique de l'acétylsalicylate de 2(o-méthoxyphénoxy)éthanol a été évaluée en comparaison avec celle de l'acide acétylsalicylique suivant la méthode, universellement employée, de la mesure de la variation de la température corporelle, augmentée par l'injection intraveineuse de nucléinate de sodium dans le lapin. Les résultats de l'élaboration statistique des épreuves sont indiqués dans le tableau 2, dans lequel l'augmentation de la température corporelle est indiquée en °C ± la déviation standard, et a été mesurée 1, 2, 3 et 4 h après l'administration; pour les autres valeurs, est valable ce qui a été spécifié en relation avec le tableau 1.

De l'examen des résultats des épreuves, indiqués dans le tableau 2, on constate que l'acétylsalicylate de 2(o-méthoxyphénoxy)-éthanol présente, passé un temps suffisant après l'administration, une activité antipyrétique supérieure à celle de l'acide acétylsalicylique.

*Activité analgésique*

L'activité analgésique de l'acétylsalicylate de 2(o-méthoxyphénoxy)éthanol a été évaluée en comparaison avec celle de l'acide acétylsalicylique sur la base de la fréquence des convulsions abdominales (writhings) induites dans les souris par une injection intrapéritonéale d'acide acétique, suivant la technique décrite par Koster et Anderson («Fed. Proc.», vol. 18, p. 412, 1959). Les résultats de l'élaboration statistique des épreuves sont indiqués dans le tableau 3, dans lequel la fréquence des convulsions est indiquée comme nombre de convulsions par minute ± la déviation standard, et a été mesurée 60 min après l'administration; pour les autres valeurs, est valable ce qui a été spécifié en relation avec le tableau 1.

De l'examen des résultats des épreuves, indiqués dans le tableau 3, on constate que l'acétylsalicylate de 2(o-méthoxyphénoxy)éthanol présente une activité analgésique nettement supérieure à celle de l'acide acétylsalicylique.

*Activité bronchosécrétolytique*

L'activité bronchosécrétolytique de l'acétylsalicylate de 2(o-méthoxyphénoxy)éthanol a été évaluée en comparaison avec celle du gaïacoléthanol, sur la base de la mesure des variations de la quantité d'indicateur (fluorescéine) trouvée dans l'arbre bronchial de rats Wistar, recueillie au moyen d'un système de lavage standardisé, après injection parentérale de l'indicateur, et mesurée au moyen d'un spectrofluoromètre, suivant la méthode décrite par H. Mawatari («Experimental studies on the expectorant action of several drugs», Kagoshima Daigakeu Igakeu Zasshi, vol. 27, p. 561, 1976). Les résultats de l'élaboration statistique des épreuves sont recueillis dans le tableau 4, dans lequel l'excrétion pulmonaire de fluorescéine est exprimée en microgramme ± la déviation standard, soit dans la quantité totale trouvée, soit dans la quantité en pour-cent rapportée à 100 g de poids corporel des rats. Entre parenthèses sont indiquées les valeurs de l'augmentation en pour-cent par rapport aux contrôles. Pour les autres valeurs, est valable ce qui a été précisé en relation au tableau 1.

De l'examen des résultats des épreuves, indiqués dans le tableau 4, on constate que l'acétylsalicylate de 2(o-méthoxyphénoxy)-éthanol présente une activité bronchosécrétolytique sensiblement supérieure à celle du gaïacoléthanol.

*Toxicité aiguë*

La toxicité aiguë de l'acétylsalicylate de 2(o-méthoxyphénoxy)éthanol a été évaluée en comparaison avec celle de l'acide acétylsalicylique et exprimée comme dose $DL_{50}$ (la dose qui provoque la mort de 50% des animaux traités) en milligrammes par kilo de poids corporel des rats auxquels la substance a été administrée par voie orale. Les résultats de l'élaboration statistique suivant la méthode décrite par J.T. Lichtfield *et al.*, («J. Pharm. Exp. Ther.», vol. 96, p. 99, 1949) donnent les valeurs suivantes de la dose toxique $DL_{50}$ et, entre parenthèses, de son intervalle fiducial (valeurs minimale et maximale relevées):

| | |
|---|---|
| acide acétylsalicylique | 1400 (1207 ÷ 1580) |
| acétylsalicylate de 2(o-méthoxyphénoxy)-éthanol | 2934 (2696 ÷ 3098) |

L'examen de ces résultats révèle que l'acétylsalicylate de 2(o-méthoxyphénoxy)éthanol présente une toxicité aiguë beaucoup moins forte que celle de l'acide acétylsalicylique.

*Toxicité subaiguë*

La toxicité subaiguë de l'acétylsalicylate de 2(o-méthoxyphénoxy)éthanol a été évaluée en comparaison avec celle de l'acide acétylsalicylique en administrant à des rats, par voie orale, des doses journalières de substance pour 10 d, et en relevant le nombre des animaux morts, la diminution moyenne de poids des animaux traités et l'incidence des lésions gastriques relevées, distinguées en simple hyperémie, hyperémie accompagnée par hémorragie et hémorragie accompagnée par ulcérations. Les résultats des épreuves sont indiqués dans le tableau 5, dans lequel la dose de substance

administrée est exprimée en milligrammes de substance par kilo de poids corporel et par jour, et la diminution moyenne de poids corporel à la fin du traitement est exprimée en grammes.

De l'examen des résultats des épreuves, indiqués dans le tableau 5, on constate que le traitement avec doses diacétylsalicylate de 2(o-méthoxyphénoxy)éthanol beaucoup plus importantes des doses correspondantes d'acide acétylsalicylique a donné lieu à un nombre beaucoup plus petit d'animaux morts, à une diminution du poids corporel beaucoup plus modéré, à une moins grande incidence d'hyperémie accompagnée par hémorragie, et à aucun cas d'hémorragie accompagnée par ulcérations. La toxicité subaiguë de l'acétylsalicylate de 2(o-méthoxyphénoxy)éthanol est donc drastiquement inférieure à celle de l'acide acétylsalicylique.

Les épreuves pharmacologiques décrites confirment donc que l'acétylsalicylate de 2(o-méthoxyphénoxy)éthanol représente un considérable progrès par rapport à une simple association d'acide acétylsalicylique et d'un dérivé du gaïacol, en relation avec l'activité globale analgésique, antipyrétique et bronchosécrétolytique et en relation avec la toxicité soit aiguë, soit subaiguë, et représente donc un agent perfectionné pour le traitement des affections fiévreuses, des maladies dérivant des rhumes, des affections rhumatismales et des affections névralgiques, et spécialement dans le traitement des affections catarrhales de l'arbre respiratoire sur base phlogistique, fiévreuse et douloureuse et des complications au niveau trachéo-broncho-pulmonaire.

La substance active selon l'invention peut être administrée par voie orale ou par voie rectale, seule ou en association avec d'autres substances (ayant une activité parallèle, synergique ou complémentaire) et à l'état pur, ou dans un véhicule ou avec un excipient pharmaceutiquement acceptable et chimiquement compatible, sous forme liquide, pâteuse, solide ou en poudre.

*Tableau 1*

| Substance | Dose (mg/kg) | Volume de l'œdème (− ml ± d.s.) | | |
|---|---|---|---|---|
| | | après 3 h | après 4½ h | après 6 h |
| = | = | 0,683 ± 0,2 | 0,608 ± 0,17 | 0,480 ± 0,2 |
| Acide acétylsalicylique | 100 | *0,380 ± 0,21 (44,0) | *0,395 ± 0,14 (35,0) | 0,285 ± 0,18 (41,0) |
| | 200 | **0,297 ± 0,17 (56,5) | *0,370 ± 0,09 (39,0) | *0,275 ± 0,18 (43,0) |
| Acétylsalicylate de 2(o-méthoxyphénoxy)éthanol | 100 | *0,380 ± 0,18 (44,0) | **0,333 ± 0,17 (45,0) | 0,291 ± 0,13 (39,0) |
| | 200 | **0,20 + 0,1 (70,0) | **0,20 + 0,11 (67,0) | *0,16 + 0,1 (65,0) |

*Tableau 2*

| Substance | Dose (mg/kg) | Augmentation température corporelle (− °C ± d.s.) | | | |
|---|---|---|---|---|---|
| | | après 1 h | après 2 h | après 3 h | après 4 h |
| = | = | 1,25 ± 0,2 | 1,45 ± 0,2 | 1,65 ± 0,22 | 0,75 ± 0,18 |
| Acide acétylsalicylique | 100 | *0,75 ± 0,16 (40) | **0,78 ± 0,17 (46) | *0,94 ± 0,12 (43) | 0,55 ± 0,1 (26) |
| | 200 | *0,62 ± 0,13 (50) | **0,71 ± 0,18 (51) | *0,93 ± 0,16 (43) | 0,52 ± 0,07 (31) |
| Acétylsalicylate de 2(o-méthoxyphénoxy)éthanol | 100 | 1,15 ± 0,19 | 1,13 ± 0,2 (22) | *0,94 ± 0,11 (43) | **0,33 ± 0,07 (56) |
| | 200 | 1,1 ± 0,2 | *1,1 ± 0,17 (24) | *0,87 ± 0,15 (47) | **0,28 ± 0,05 (62) |

*Tableau 3*

| Substance | Dose (mg/kg) | Fréquence (writhings) | % inhibition |
|---|---|---|---|
| = | = | 29,22 ± 8,0 | = |
| Acide acétylsalicylique | 100<br>200 | *20,5 ± 6,7<br>**16,4 ± 5,8 | 30<br>44 |
| Acétylsalicylate de 2 (o-méthoxyphénoxy)éthanol | 100<br>200 | **19,5 ± 5,4<br>**14,88 ± 6,8 | 33<br>49 |

*Tableau 4*

| Substance | Dose (mg/kg) | Excrétion pulmonaire de fluorescéine | |
|---|---|---|---|
| | | Quantité totale (µg) | Quantité % (µg/100 g) |
| = | = | 0,252 ± 0,047 | 0,119 ± 0,026 |
| Gaïacoléthanol | 101,8 | **0,434 ± 0,064 (+72,2) | 0,202 ± 0,034 |
| Acétylsalicylate de 2 (o-méthoxyphénoxy)éthanol | 200 | **0,481 ± 0,068 (+90,8) | 0,230 ± 0,042 |

*Tableau 5*

| Substance | Dose (mg/kg) die | Nombre animaux traités | Nombre animaux morts | Δ Poids (moyenne) | Lésions gastriques | | |
|---|---|---|---|---|---|---|---|
| | | | | | Hyperémie | Hyperémie + hémorragie | Hémorragie + ulcérations |
| Acide acétylsalicylique | 500 | 10 | 6 | −30 | 4 | 3 | 2 |
| Acétylsalicylate de 2(o-méthoxyphénoxy)éthanol | 915 | 10 | 3 | −10 | 4 | 2 | = |

**Revendications**

1. Dérivé de l'acide acétylsalicylique, constitué par acétylsalicylate de 2(o-méthoxyphénoxy)-éthanol, avec la formule suivante structurale:

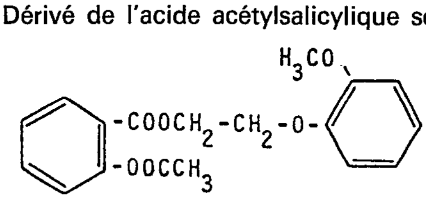

2. Dérivé de l'acide acétylsalicylique selon la revendication 1, indiqué pour l'utilisation comme agent ayant une activité analgésique, antipyrétique, antiphlogistique et bronchosécrétolytique.

3. Dérivé de l'acide acétylsalicylique selon la revendication 1, indiqué pour l'utilisation dans le traitement des affections fiévreuses, des maladies dérivant des rhumes, des affections rhumatismales et des affections névralgiques, et spécialement dans le traitement des affections catarrhales de l'arbre respiratoire sur base phlogistique, fiévreuse et douloureuse, et des complications au niveau trachéo-broncho-pulmonaire.

## Patentansprüche

1. Derivat der Acetylsalicylsäure, bestehend aus 2-(o-Methoxyphenoxy)äthanolacetylsalicylat mit der folgenden Strukturformel:

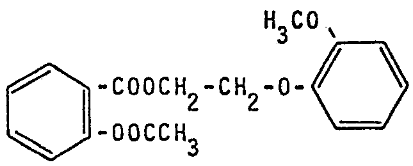

2. Derivat der Acetylsalicylsäure nach Anspruch 1, geeignet zur Verwendung als Mittel mit analgetischer, antipyretischer, antiphlogistischer und bronchosekretolytischer Aktivität.

3. Derivat der Acetylsalicylsäure nach Anspruch 1, geeignet zur Verwendung in der Behandlung von fiebrigen Affektionen, Erkältungskrankheiten, rheumatischen und neuralgischen Affektionen, und insbebesondere in der Behandlung der katarrhalischen Affektionen des Atmungsapparats auf entzündlicher, fiebriger und schmerzhafter Basis, und der Komplikationen auf dem tracheo-broncho-pulmonalen Niveau.

## Claims

1. A derivative of acetylsalicylic acid, formed out of 2-(o-methoxyphenoxy)ethanol acetylsalicylate with the following structural formula:

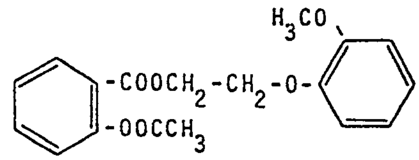

2. A derivate of acetylsalicylic acid as claimed in Claim 1, suitable for use as an agent having analgetic, antipyretic, antiphlogistic and bronchosecretolytic activity.

3. A derivate of acetylsalicylic acid as claimed in Claim 1, suitable for use in the treatment of feverish affections, chill diseases, rheumatic and neuralgic affections, and especially in the treatment of the catarrhal affections of the respiratory apparatus on a phlogistic, feverish and painful basis, as well as of the complications at the tracheo-broncho-pulmonary level.